# EUROPÄISCHE PATENTSCHRIFT

(11) **EP 3 592 269 B1**
(45) Veröffentlichungstag und Bekanntmachung des Hinweises auf die Patenterteilung: **23.06.2021**
(21) Anmeldenummer: 18710821.2
(22) Anmeldetag: 07.03.2018
(51) Int. Cl.: A61B 18/14, A61B 17/072, A61B 17/29

(54) **CHIRURGISCHES INSTRUMENT MIT EINEM KOPPELMECHANISMUS ZUM ANTREIBEN EINES SCHNEIDELEMENTS**
SURGICAL INSTRUMENT WITH A COUPLING MECHANISM FOR DRIVING A CUTTING ELEMENT
INSTRUMENT CHIRURGICAL POURVU D'UN MÉCANISME D'ACCOUPLEMENT AUX FINS D'ENTRAÎNEMENT D'UN ÉLÉMENT DE COUPE

(30) Priorität: 08.03.2017 DE 102017104849
(43) Veröffentlichungstag der Anmeldung: 15.01.2020
(73) Patentinhaber: Aesculap AG, 78532 Tuttlingen (DE)
(72) Erfinder: WALBERG, Erik, 78532 Tuttlingen (DE); HERNER, Eugen, 78054 Villingen-Schwenningen (DE)
(74) Vertreter: Winter, Brandl - Partnerschaft mbB
(86) Internationale Anmeldenummer: PCT/EP2018/055634
(87) Internationale Veröffentlichungsnummer: WO 2018/162569

(56) Entgegenhaltungen:
- EP-A1- 2 845 551
- EP-A2- 1 621 142
- CN-U- 204 581 463
- US-A1- 2011 087 208
- US-A1- 2016 074 105

## Beschreibung

Die vorliegende Erfindung betrifft ein chirurgisches Instrument, etwa nach Art eines bipolaren Seal & Cut (S&C) Instruments der TFT-Bauart (TFT steht für "THERMAL FUSION TECHNOLOGY"), also eines Verschließ-und-Schneide-Instruments, gemäß dem Oberbegriff des Anspruchs 1. Jenes chirurgische Instrument weist einen Schaftabschnitt auf, der an seinem distalen Endbereich ein axial verfahrbares Schneidelement zumindest teilweise beherbergt/lagert. Weiterhin ist an dem chirurgischen Instrument ein Handgriff angeordnet, in dem ein Koppelmechanismus eine von einem Anwender, wie einem Chirurgen, durchgeführte Auslösebewegung an einem Auslöseelement (Handhabe) in eine axiale Schnittbewegung des Schneidelements umwandelt.

### Hintergrund der Erfindung

In der minimalinvasiven Chirurgie sowie der Endoskopie spielt die Bauraum- und Gewichtsoptimierung eine wichtige Rolle. So ist nicht nur an dem distalen Instrumentenkopf, sondern auch an dem vom Anwender betätigten proximalen Instrumentengriff auf eine platz- und gewichtssparende Ausgestaltung zu achten.

Gattungsgemäße chirurgische Instrumente weisen in ihrem proximalen Instrumentengriff einen Mechanismus zum Hervorrufen eines Verfahrweges des Schneidelements, den sogenannten Koppelmechanismus, auf, damit mit dem Schneidelement ein linearer Schnitt an einem Patienten durchführbar ist. Der Koppelmechanismus wandelt die von dem Auslöseelement hervorgerufene Bewegung, vorzugsweise in Form einer Rotation, demnach in eine lineare Bewegung, also eine Axialbewegung, des Schneidelements um.

Hierbei ist eine reibungsarme, kompakte sowie zuverlässige Kraftübertragung von dem Auslöseelement auf das Schneidelement essentiell, um den hohen Anforderungen an die Präzision und Lebensdauer von chirurgischen Instrumenten gerecht zu werden.

### Stand der Technik

Aus der Deutschen Gebrauchsmusterschrift DE 20 2004 010 313 U1 ist ein chirurgisches Instrument mit einem Handgriff bekannt, an dem ein Auslöseelement angeordnet ist. Die von einem Anwender hervorgerufene Teilrotation des Auslöseelements wird über einen nicht näher beschriebenen Koppelmechanismus in eine Teilrotation um eine andere Achse gewandelt.

Weiterhin sind aus dem Stand der Technik chirurgische Instrumente mit Koppelmechanismen bekannt, die von einem Hebel oder einem Nocken betätigbar sind und mittels eines herkömmlichen Getriebes oder eines Sperrklinkenmechanismus eine Linearbewegung eines Schneidelements hervorrufen. Hierbei sind meist mehrere gleichartige Betätigungen des Auslöseelements nötig, um einen vollständigen Schnitt durchzuführen.

Weitere Koppelmechanismen sind mittels einer Ritzel-Zahnstangen-Kombination realisiert. Diese sind besonders vorteilhaft, um eine Rotation zuverlässig in eine Translation zu übertragen. Hierbei ist das Auslöseelement entweder mit einer ersten Zahnstange oder direkt mit dem starr im Handgriff angeordneten Ritzel gekoppelt, um auf eine weitere Zahnstange eine Bewegung zu übertragen. Die weitere Zahnstange ist wiederum starr mit dem Schneidelement gekoppelt.

Nachteilig an dem Stand der Technik ist, dass mit zunehmender Länge des von dem Schneidelement durchzuführenden Schnitts mehr Platz durch den Koppelmechanismus in dem Instrumentengriff eingenommen wird, da der größtmögliche Verfahrweg des Schneidelements in etwa der Länge des Koppelmechanismus entspricht. Dies läuft dem Ziel der Bauraum- und/oder Gewichtsoptimierung zuwider.

Weiterer gattungsgemäßer Stand der Technik ist aus den Dokumenten DE 20 2004 010 313 U1, DE 10 2010 016 291 A1 und EP 2 845 551 A1 bekannt.

### Kurzbeschreibung der Erfindung

Der vorliegenden Erfindung liegt angesichts dieses Standes der Technik die Aufgabe zugrunde, die Nachteile aus dem Stand der Technik zu beheben oder zumindest zu mildern, und insbesondere ein chirurgisches Instrument der gattungsgemäßen (insbesondere nach dem TFT-Prinzip arbeitenden) Art zu offenbaren, das lange / weite Verfahrwege des Schneidelements, also Schnitte, ermöglicht, ohne entsprechend mehr Bauraum und/oder Gewicht in Anspruch zu nehmen.

Dies wird erfindungsgemäß mittels eines chirurgischen Instruments mit den Merkmalen des Anspruchs 1 gelöst. Vorteilhafte Ausführungsformen sind Gegenstand der Unteransprüche.

Aus dieser erfindungsgemäßen Ausgestaltung des chirurgischen Instruments lassen sich beispielsweise folgende weitere Vorteile ableiten:
- Ohne ein zusätzliches Zahnrad wird die Übersetzung zwischen dem Auslöseelement und dem Ritzel erhöht.
- Die Rotation des Ritzels resultiert aus einer (handgriff)-festen Zahnstange, die flexibel anbringbar ist, was die Anpassbarkeit des erfindungsgemäßen Koppelmechanismus erhöht.
- Der Hub einer Zahnstange lässt sich aus dem Produkt der Kreiszahl Pi sowie dem Durchmesser und der Drehzahl des Ritzels bestimmen, wobei die Zahnstange ungefähr so lange wie ein Hub sein sollte. Erfindungsgemäß wird demnach bei gleichbleibendem Durchmesser des Ritzels und auch bei gleichbleibender Drehzahl der Hub verdoppelt, wobei die Zahnstange nur halb so lange wie der Hub sein muss. Alternativ kann bei konstantem Hub der benötigte Bauraum und die benötigte Schwenkbewegung am Auslöseelement (Handhabe) signifikant reduziert werden.

Der Gegenstand der Erfindung ist demnach ein chirurgisches Instrument mit einem Schaftabschnitt, der an seinem distalen Ende/Endbereich ein axial verfahrbares Schneidelement, wie etwa eine Klinge / ein Messer / eine HF-Schneideinrichtung / etc., das über eine in dem Schaftabschnitt gelagerte (Zug-/Druck-)Stange angebunden/betätigbar ist, zumindest teilweise beherbergt/lagert. Das chirurgische Instrument hat weiter einen den Schaftabschnitt (an dessen proximalem Ende/Endbereich) aufnehmenden Handgriff, an dem ein etwa hebelartiges Auslöseelement (Handhabe) für das Schneidelement beweglich angeordnet ist, sowie in dem ein Koppelmechanismus untergebracht ist, der eine (Teil-)Drehung des vorzugsweise hebelartigen Auslöseelements unter Zwischenschaltung eines Ritzels oder einer Reibrolle in eine Axialbewegung des Schneidelements über die (Zug-/Druck)-Stange wandelt.

Gemäß der Erfindung ist der Koppelmechanismus so konstruiert, dass die Drehung/Schwenkbewegung des Auslöseelements um dessen Schwenkachse sowohl eine Rotation des Ritzels/der Reibrolle um seine Mittelachse und zusätzlich eine Translations-/Axialbewegung der Mittelachse des Ritzels/der Reibrolle (in dessen/deren Rotationsrichtung) bewirkt. Diese beiden Bewegungs-Komponenten, nämlich die Rotation des Ritzels/der Reibrolle um die Mittelachse und die Translation des Ritzels/der Mittelachse addieren sich, um den Verfahrweg des Schneidelements insgesamt zu verlängern. Der aus der Rotation des Ritzels/der Reibrolle resultierende Verfahrweg ist über den Durchmesser des Ritzels/der Reibrolle variierbar. Der aus der Translation der Mittelachse des Ritzels/der Reibrolle resultierende Verfahrweg ist über die Länge und den Drehpunkt des Auslöseelements (relevante/aktive Hebellänge) variierbar. Somit ist das chirurgische Instrument mit einer Vielzahl von Komponenten / Parametern / Stellmöglichkeiten ausgestattet, um auf den Verfahrweg des Schneidelements bei kleinstmöglichem Bauraum- sowie Gewichtsbedarf in diesem verlängernder oder verkürzender Weise Einfluss zu nehmen.

In anderen Worten ausgedrückt lässt sich das vorstehende Funktionsprinzip konstruktiv beispielsweise dadurch umsetzen, indem das handgriffseitige Auslöseelement (Handhabe) als ein zentral (irgendwo mittig) schwenkbar gelagerter (Betätigungs-) Hebel ausgebildet ist mit einem am freien Ende des hebelförmigen Auslöseelements (gegenüber dem Betätigungsende des Auslöseelements) frei (in Hebellängsrichtung verschiebbar) gelagerten Ritzel/Reibrolle, das wiederum zwischen einer unbeweglichen (z. B. am Griffgehäuse fixierten) Abwälzlaufbahn, wie fixe Zahnstange, und einer vorzugsweise parallel dazu beweglichen Abwälzlaufbahn, wie (parallel-)verschiebbare Zahnstange, eingeklemmt ist. Wird der Hebel geschwenkt und damit die Mittelachse des Ritzels/der Reibrolle zwischen den beiden Zahnstangen translatorisch bewegt, wälzt das Ritzel/die Reibrolle an der fixen Abwälzlaufbahn ab und treibt dabei in einem bestimmten Übersetzungsverhältnis entsprechend der aktiven Hebelarmlänge und dem Ritzel-/Reibrollendurchmesser die bewegbare Abwälzlaufbahn an, die mit dem Schneidelement über die (Zug-/Druck-) Stange gekoppelt ist. Durch die Zwischenschaltung des Ritzels/der Reibrolle zwischen dem (manuell betätigbaren) Auslöseelement und der (Zug-/Druck-)Stange kann bei gleichbleibendem Auslöse-/Betätigungsweg des Auslöseelements (Schwenkwinkel) der Verschiebeweg der (Zug-/Druck-)Stange variiert werden, in dem der Ritzel-/Reibrollen-Durchmesser verändert wird.

Grundsätzlich kann eine Verlängerung des Verschiebewegs der (Zug-/Druck-)Stange natürlich dadurch erreicht werden, indem der Hebelarm des Auslöseelements einfach verlängert wird. Dies würde aber dazu führen, dass das Griffgehäuse größer bauen muss, um das verlängerte Auslöseelement darin (teilweise) aufzunehmen. Durch die Zwischenschaltung des Ritzels/der Reibrolle kann jedoch eine zusätzliche Übersetzung in den Kopplungs-/Koppelmechanismus integriert werden, wodurch eine Verlängerung des Auslöseelements vermeidbar ist. Dadurch ist es auch nicht mehr erforderlich, das Griffgehäuse (wesentlich) zu vergrößern.

In einer bevorzugten Ausführungsform überschreitet die Axialbewegung des Schneidelements, also die Länge des Verfahrwegs bzw. die Länge des Schnitts an einem Patienten, die von der Mittelachse des Ritzels durchgeführte, von dem Auslöseelement unmittelbar hervorgerufene Axialbewegung. So ist es dem Anwender ermöglicht, mittels einer kurzen Betätigungsbewegung einen verglichen dazu langen Schnitt hervorzurufen. Neben den vorstehend erwähnten Vorteilen wirkt sich dies positiv auf die von Seiten des Anwenders aufzubringende Kraft aus. Denn häufig dauern Operationen mehrere Stunden, weshalb jede Krafteinsparung auf Seiten des Anwenders / Chirurgen, die aus einer Komforterhöhung der Bedienung resultiert, das Fehlerrisiko minimiert und somit auch für sicherheitstechnische Aspekte relevant ist.

Insbesondere dadurch, dass ein Zahnbereich des Ritzels in eine (handgriff)-feste/fixe Zahnstange eingreift, während zeitgleich ein anderer/gegenüberliegender Zahnbereich des Ritzels in eine schneidelementfeste/parallelverschiebbare/relativ zum Handgriff bewegliche Zahnstange eingreift, ist eine hohe Kompaktheit des Instruments erreicht.

Eine vorteilhafte erfindungsgemäße Ausgestaltung der vorliegenden Erfindung zeichnet sich dadurch aus, dass die Axialbewegung des Schneidelements im Wesentlichen doppelt so groß ist wie die von der Mittelachse des Ritzels durchgeführte Axialbewegung. Demnach sind der Durchmesser des Ritzels sowie das von dem ritzelseitigen Ende des Auslöseelements bei dessen Betätigung abgefahrene Kreisbogensegment derart aufeinander abgestimmt, dass der Betätigungsweg des Auslöseelements, also die Bewegung, die der Anwender für einen Schnitt auszuführen hat, halb so groß ist, wie der eigentliche Schnitt. Dieses Verhältnis von im Wesentlichen 1:2 trägt dem Ziel der Bauraumoptimierung sowie der Kraftreduzierung ausreichend Rechnung, während es weiterhin eine solche Bedienbarkeit des Instruments bewirkt, dass der Anwender intuitiv ein Gefühl für die Länge des Schnitts hat.

Insbesondere dann, wenn die von der Mittelachse des Ritzels durchgeführte Axialbewegung im Wesentlichen der Axialbewegung eines Griffbereichs des Auslöseelements entspricht, ist eine kompakte Anordnung des Koppelmechanismus gewährleistet. Der Griff-/Betätigungsbereich des (hebelförmigen) Auslöseelements befindet sich am (bezüglich dessen mittige Lagerung) unteren, also ritzelfernen Ende des Auslöseelements. Wenn seine Axialbewegung, oder anders ausgedrückt der axiale Anteil seiner Rotationsbewegung, im Wesentlichen der Axialbewegung der Mittelachse des Ritzels entspricht, bedeutet das, dass der Punkt, um den aus Auslöseelement rotiert, mittig angeordnet ist. Jener Rotationspunkt des Auslöseelements ist bevorzugt an einem Rand des Handgriffs (nahe dem Griffgehäuse) ausgeformt. Somit ist im Wesentlichen die eine Hälfte des Auslöseelements im Inneren des Handgriffs angeordnet, während die andere Hälfte außerhalb des Handgriffs angeordnet ist. Das Auslöseelement weist auf diese Weise weder in die eine, noch in die andere Richtung einen Überhang auf, was sich positiv auf dessen Dynamik auswirkt.

In einer vorteilhaften Ausführungsform der Erfindung greift der Zahnbereich des Ritzels, der in die feste Zahnstange eingreift, nach einer halben Umdrehung des Ritzels in die bewegliche Zahnstange ein. Anders ausgedrückt greift derselbe Zahnbereich abwechselnd in die ortsfeste feste Zahnstange und die vom Ritzel angetriebene bewegliche Zahnstange ein. Hieraus folgt, dass zu dem Zeitpunkt, zu dem ein Zahnbereich in die eine Zahnstange eingreift, ein anderer in die andere Zahnstange eingreift. Somit werden auf minimalem Bauraum um das Ritzel herum verschiedene Zahneingriffe realisiert, was den Bauraumbedarf des Koppelmechanismus senkt. Hierbei ist je nach Ausführungsform die Längsachse der jeweiligen Zahnstangen in ihrer Ausrichtung variierbar, solange sie tangential zum Ritzel verlaufen (und auch bevorzugt parallel zueinander ausgerichtet sind).

Gemäß der Erfindung weist das Ritzel entlang seiner Mittelachse zwei konzentrische Abschnitte unterschiedlichen Durchmessers auf, bei dem der eine Abschnitt in die feste Zahnstange eingreift, während der andere Abschnitt in die bewegliche Zahnstange eingreift. Aus den unterschiedlichen Durchmessern folgt eine zusätzliche Übersetzungsstufe des Koppelmechanismus. Je nach Anwendungsfall ist der jeweilige Abschnitt flexibel auf die herrschenden Anforderungen anpassbar. Somit ist die Durchmesserabweichung der jeweiligen Abschnitte frei ausgestaltbar.

Insbesondere vorteilhaft ist es, wenn das Ritzel in der Höhenrichtung des chirurgischen Instruments (bzw. im Wesentlichen längs des hebelförmigen Auslöseelements) relativ zu dem Auslöseelement frei beweglich ist, während es in der Axialrichtung des Schafts/Schaftabschnitts (bzw. quer zum hebelförmigen Auslöseelement) fix mit dem Auslöseelement gekoppelt ist. Die freie Beweglichkeit in der Höhenrichtung ist mittels eines gabelförmigen Lagerbocks (längs des hebelförmigen Auslöseelements) realisiert, der zwei schlitzartige Ausnehmungen (Lagergabeln) zur Aufnahme der Mittelachse des Ritzels aufweist. Mittels der sich gegenüber liegenden länglichen Schlitze ist eine Relativbewegung in der vorstehend definierten Höhenrichtung realisiert. Dadurch, dass die Breite der Schlitze im Wesentlichen dem Durchmesser der Mittelachse des Ritzels entspricht (geringfügig weiter), ist die fixe Kopplung der Mittelachse des Ritzels in der Axialrichtung sichergestellt. So ist auf engstem Bauraum eine Lagerung des Ritzels am hebelförmigen Auslöseelement mit zwei Freiheitsgraden - nämlich der Rotation um die Mittelachse des Ritzels und die Translation des Ritzels in der Höhenrichtung (d.h. längs des hebelförmigen Auslöseelements) - ermöglicht.

Sobald die feste Zahnstange integral mit dem Handgriff ausgestaltet ist, ist die Herstellung des Handgriffs, die vorteilhafterweise über ein Spritzgussverfahren erfolgt, zeit- und kosteneffizient realisiert. Alternativ ist es ebenfalls möglich, die feste Zahnstange als separates Bauteil zu fertigen und etwa über einen Stoffschluss (oder durch Niete oder Clipse) mit dem Handgriff zu verbinden. Dies erhöht die Modularität des chirurgischen Instruments

In einer weiteren vorteilhaften Ausführungsform der vorliegenden Erfindung weist das Ritzel Verzahnungen verschiedenen Durchmessers / verschiedener Geometrie auf, sodass mittels einer Schaltbewegung des Ritzels entlang seiner Mittelachse die Axialbewegung des Schneidelements variierbar ist. Wie vorstehend erläutert, variiert die von dem Ritzel auf die bewegliche Zahnstange und somit auf das Schneidelement übertragene lineare Bewegung mit dem Durchmesser des Ritzels. Mittels der Ausgestaltung von verschiedenen Verzahnungsgeometrien, die wenn sie eingreifen, jeweils in beiden Zahnstangen eingreifen, gepaart mit der Schaltbewegung sind somit verschiedene Übersetzungsstufen auf in Höhen- und Axialrichtung des Griffs gleichem Bauraum verwirklicht. Mittels dieser Schaltbarkeit verschiedener Ritzelgrößen nimmt die Flexibilität des chirurgischen Instruments weiter zu.

Weitergehend sei erwähnt, dass der Schaftabschnitt des erfindungsgemäßen chirurgischen Instruments in einer Ausführungsform dazu vorbereitet ist, eine Drehbewegung um seine Längsachse durchzuführen. Jene Drehbewegung wird etwa durch den Koppelmechanismus gelagert.

In einer Ausführungsform gemäß der Erfindung des chirurgischen Instruments erstreckt sich die bewegliche Zahnstange einerseits in der Axialrichtung des Schaftabschnitts und andererseits zumindest teilweise in Umfangsrichtung um den Schaftabschnitt. Dies ermöglicht bei der vorstehend genannten Drehbewegung des Schaftabschnitts um seine Längsachse, dass das Ritzel weiterhin in die bewegliche Zahnstange eingreift. Die Drehbewegung kann sich in einer Winkelspanne von bis zu 360° erstrecken. Somit ist eine hochflexible Schneidebewegung ermöglicht. Dadurch, dass bei jener Rotation das Ritzel weiterhin in Kontakt mit der bewegliche Zahnstange ist, weist das chirurgische Instrument einen robusten Rotationsübertragungsmechanismus auf.

Insbesondere dann, wenn zwischen dem Auslöseelement und dem Ritzel eine zusätzliche Koppeleinheit angeordnet ist, über die eine Relativverschiebung zwischen dem Koppelmechanismus und dem Schneideelement bei unveränderter Stellung des Auslöseelements ermöglicht ist, lässt sich eine auf das jeweilige Anwendungsgebiet maßgeschneiderte Schneidebewegung realisieren. Durch das Betätigen der zusätzlichen Koppeleinheit verschiebt sich somit das Schneidelement relativ zum Koppelmechanismus bei unveränderter Stellung des Auslöseelements.

Vorteilhaft ist es auch, wenn der Koppelmechanismus eine Rückholfeder, vorzugsweise nach Art einer Zugfeder, einer Druckfeder oder einer Torsionsfeder, beherbergt, die dazu vorbereitet ist, das Schneidelement nach einer Betätigung in seine Ausgangsposition zurückzuversetzen. Somit ist von Seiten des bedienenden Chirurgen keine Kraft aufzubringen, um nach einer Schneidebewegung das Auslöseelement und das Schneidelement in die Ausgangsstellung zu bringen. Im Falle der Ausgestaltung mittels einer Zugfeder ist diese an der beweglichen Zahnstange, dem Ritzel oder dem Auslöseelement im Bereich des Ritzels angeordnet. Im Falle der Ausgestaltung mittels einer Druckfeder ist diese distal zur beweglichen Zahnstange, dem Ritzel oder dem Auslöseelement im Bereich des Ritzels angeordnet. Weiterhin kann die Druckfeder auch um den Schaftabschnitt herum angeordnet sein. Eine weitere Ausgestaltung der Rückholfeder besteht in einer Torsionsfeder. Diese ist vorzugsweise um die Rotationsachse des Auslöseelements herum anzuordnen, um auf diese im Falle eines Rückholens des Schneidelements eine Kraft auszuüben.

Der erfinderische Gedanke umfasst überdies die Ausgestaltung des erfindungsgemäßen Ritzels in einem herkömmlichen Scheren-Greif-Instrument, in dem ein kleiner Hebel oder ein Gleitelement das Ritzel antreibt, um das Schneidelement zu bewegen.

Vorteilhafterweise sind die zwei konzentrischen Verzahnungsabschnitte des Ritzels integral mit- bzw. zueinander ausgestaltet. Alternativ hierzu sind die zwei konzentrischen Verzahnungsabschnitte als zwei zueinander separate Bauteile ausgestaltet, die weiter bevorzugt durch einen Spalt voneinander getrennt sind.

Im Rahmen der Erfindung ist es vorteilhafterweise angestrebt, dass der Verzahnungsabschnitt mit dem größeren Durchmesser in die feste Zahnstange eingreift, während der Verzahnungsabschnitt mit dem kleineren Durchmesser in die bewegliche Zahnstange eingreift. So wird erreicht, dass die Umlaufbewegung über einen ersten Winkelabschnitt des größeren Zahnrads / Verzahnungsabschnitts auf eine vergrößerte Umlaufbewegung eines zweiten Winkelabschnitts des kleineren Zahnrads / Verzahnungsabschnitts übertragen wird, wodurch sich diese Ausführungsform insbesondere für besonders lange Schäfte eignet.

Nachfolgend sei die Erfindung anhand eines bevorzugten Ausführungsbeispiels unter Bezugnahme auf die begleitenden Figuren näher erläutert. Es zeigen:
- Fig. 1:: eine Schnittansicht durch einen Handgriff entlang der Axialrichtung eines erfindungsgemäßen chirurgischen Instruments in einem ersten Zustand;
- Fig. 2:: den Handgriff aus Fig. 1 in einem Zustand, in dem ein Auslöseelement teilweise betätigt worden ist;
- Fig. 3:: den Handgriff aus Fig. 1 in einem Zustand, in dem das Auslöseelement vollständig betätigt worden ist; und
- Fig. 4:: eine schematische Ansicht eines Ritzels mit zwei Verzahnungsabschnitten.

Die Figuren sind lediglich schematischer Natur und dienen ausschließlich dem Verständnis der Erfindung. Die gleichen Elemente sind mit denselben Bezugszeichen versehen.

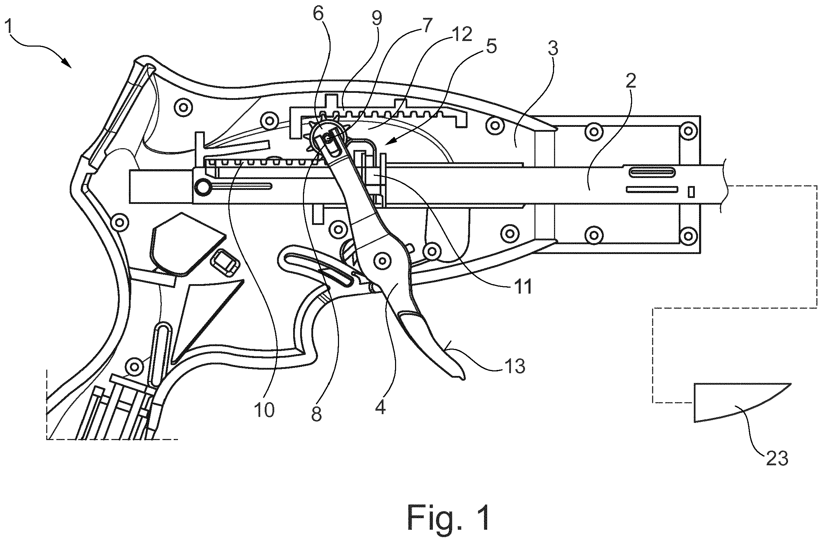

Fig. 1 zeigt ein chirurgisches Instrument 1 beispielsweise der minimal invasiven Bauart mit einem sich in Richtung distal erstreckenden Schaftabschnitt (Instrumentenschaft) 2, der an seinem distalen Ende ein axial verfahrbares Schneidelement 23 zumindest teilweise beherbergt. Das Schneidelement 23 und seine Kopplung an den Schaftabschnitt 2 ist lediglich schematisch dargestellt. In der realen Ausführungsform ist das Schneidelement 23 starr und geradlinig gekoppelt. Das Instrument 1 weist einen pistolenartigen Handgriff 3 auf, an dem ein hebelartiges Auslöseelement 4, über das das Schneidelement 23 betätigbar ist, drehbar angebracht ist. Das von einem Anwender, wie einem Chirurgen, über einen Griffbereich 13 (des Auslöseelements 4) betätigbare Auslöseelement 4 ist an seinem dem Griffbereich 13 abgewandten Ende mit einem Kopplungs-/Koppelmechanismus 5 wirkverbunden. Der Koppelmechanismus 5 wandelt die vom Anwender hervorgerufene Teildrehung des Auslöseelements 4 unter Zwischenschaltung eines Ritzels 6 in eine lineare Bewegung / eine Axialbewegung des Schneidelements 23 um.

Hierfür weist der Koppelmechanismus 5 erfindungsgemäß zwei Zahnstangen, nämlich eine (handgriff)-feste Zahnstange 9 und eine relativ zum Handgriff bewegliche (und damit verschiebbare) Zahnstange 10, auf, die parallel zueinander ausgerichtet sind. Das Ritzel 6 greift in der vorliegenden Ausführungsform mit einem ersten, oberen Zahnabschnitt in die feste Zahnstange 9 ein, während sie mit einem zweiten, unteren Zahnabschnitt in die bewegliche Zahnstange 10 eingreift.

Um dem Ritzel 6 bei gleichzeitigem Eingriff in zwei verschiedene Zahnstangen 9, 10 eine Rotation zu ermöglichen, ist die Mittelachse 7 des Ritzels 6 in Axialrichtung (d.h. längs der Zahnstangen 9, 10) beweglich ausgestaltet. Hieraus folgt, dass eine Betätigung des Auslöseelements 4 eine Rotation desselben um dessen Lagerpunkt hervorruft, die wiederum das Ritzel 6, das mit dem Auslöseelement 4 wirkverbunden/gekoppelt ist, zum einen in Axialrichtung bewegt, zum anderen, unter Zusammenwirkung mit den jeweiligen Zahnstangen 9, 10, um seine Mittelachse 7 rotiert.

Der erfindungsgemäße Koppelmechanismus/-bereich 5 ist somit derart ausgestaltet, dass die Drehung des Auslöseelements 4 eine Rotation des Ritzels 6 um seine Mittelachse 7 und zusätzlich eine Axialbewegung der Mittelachse 7 bewirkt.

Die feste Zahnstange 9 ist ortsfest angeordnet, woraus eine Rotation des Ritzels 6 in Richtung des Pfeils 12 resultiert. Die lineare Bewegung der beweglichen Zahnstange 10 setzt sich aus zwei Anteilen zusammen:
- Der erste Anteil resultiert aus der Axialbewegung der Mittelachse 7 des Ritzels 6, die ausschließlich von dem Auslöseelement 4 hervorgerufen wird.
- Der zweite Anteil resultiert aus der Rotation 12 des Ritzels 6, die die bewegliche Zahnstange 10 antreibt. Jene Rotation 12 wird von der ortsfesten festen Zahnstange 9 in Zusammenspiel mit der Axialbewegung der Mittelachse 7 erzeugt.

Im vorliegenden Ausführungsbeispiel sind die beiden Anteile etwa gleichgewichtet.

Der erste Anteil wird durch die Winkelspanne bestimmt, die der Griffbereich 13 von einem Anwender ausgelöst zurücklegt. Jene Winkelspanne hängt, abgesehen von der Bewegung des Anwenders, mit der Länge des Auslöseelements 4 und dessen Lagerpunkt zusammen. Der Lagerpunkt ist in einem Randbereich des Handgriffs 3 ausgestaltet und etwa mittig entlang der Längsachse des hebelartigen Auslöseelements 4 angeordnet.

An dem dem Griffbereich 13 abgewandten Ende des Auslöseelements 4 ist ein (gabelförmiger) Lagerbock 8 ausgestaltet. Dieser sorgt einerseits für die axiale Führung der Mittelachse 7, andererseits ermöglicht er aufgrund seiner länglichen Schlitze, dass die Rotation des Auslöseelements eine lineare Bewegung der Mittelachse 7 hervorruft. Das heißt, der Lagerbock 8 ist dergestalt, dass er zwei Freiheitsgrade der Mittelachse 7 zulässt, nämlich den in der Höhenrichtung des chirurgischen Instruments 1 und die Rotation entlang der Mittelachse 7. Jene Rotation wird vorzugsweise über eine zusätzliche Lagerung der Mittelachse 7 sichergestellt, um eine gleichmäßige, verschleißarme Rotation zu garantieren.

Ein Verbindungsteil 11 stellt eine Verbindung zwischen der beweglichen Zahnstange 10 und dem am distalen Ende angeordneten Schneidelement 23 her. Das Verbindungsteil 11 ist bügelartig ausgestaltet und verbindet vorzugsweise die bewegliche Zahnstange 10 mit einer (Zug-/Druck-)-Stange/Betätigungsstange, die wiederum das Schneidelement 23 starr führt. Jene Betätigungsstange verläuft bevorzugt im Inneren des Schaftabschnitts 2.

Das Verbindungsteil 11 kann auch als Cutter Collar oder Schneidekragen bezeichnet werden. Es beherbergt vorzugsweise ein Radiallager, um eine mögliche Rotation des Schneidelements um die Längsachse des Schaftabschnitts zu ermöglichen.

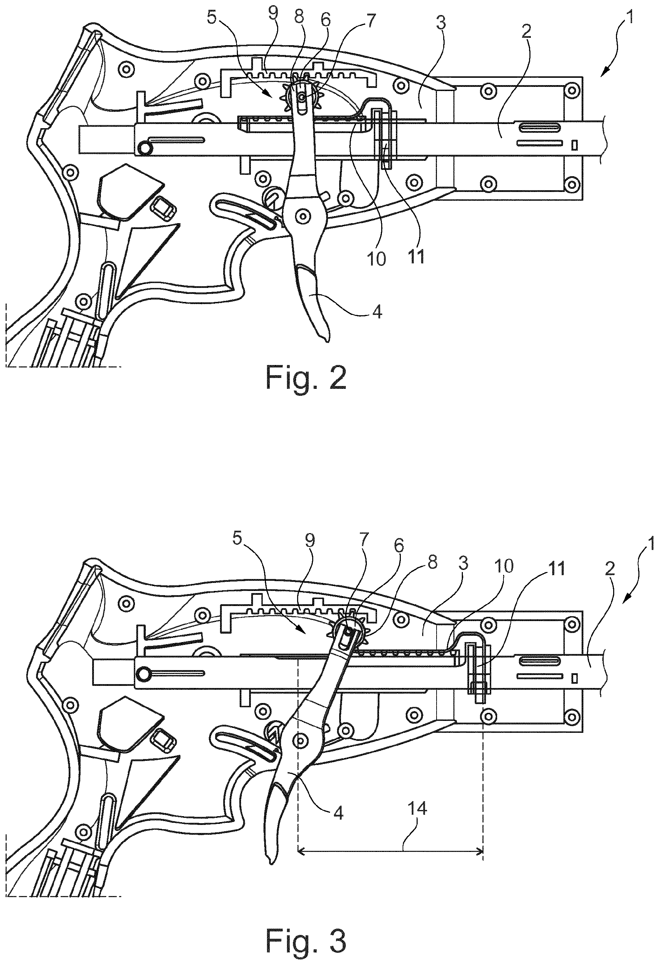

In Fig. 2 ist das chirurgische Instrument 1 in einem zweiten Zustand dargestellt. Auf die schematische Ansicht des Schneidelements 23 ist hier ebenso wie in Fig. 3 verzichtet. Das Auslöseelement 4 ist an seinem Griffbereich 13 um eine gewisse Winkelspanne um seinen Lagerpunkt in Richtung proximal verschwenkt worden. Auf der gegenüberliegenden Seite des Lagerpunktes bewirkt dies eine Bewegung der Mittelachse 7 des Ritzels 6 in Richtung distal. Steht das Ritzel in dem vorliegenden Zustand etwa mittig in der festen Zahnstange 9. Hieraus folgt, dass das Schneidelement 23 in etwa die Hälfte seines Hubes zurückgelegt hat.

Dem erfinderischen Gedanken Folge leistend, hat das Verbindungsteil 11 in axialer Richtung eine größere Distanz zurückgelegt als die Mittelachse 7. Dies folgt daraus, dass das Ritzel 6 neben der Axialbewegung seiner Mittelachse 7 nun etwa mittig in die bewegliche Zahnstange 10 eingreift, während es in Fig. 1 noch an dessen distalen Ende war. Die zurückgelegte Distanz des Schneidelements 23 setzt sich also aus der der Mittelachse 7 addiert mit der Distanz der Relativposition zwischen dem Ritzel 6 und der beweglichen Zahnstange 10, die auch als Cutter Rack bezeichnet wird.

Um die beiden verschiedenen Antriebe der festen Zahnstange 9, also die Axialbewegung der Mittelachse 7 und die Rotation des Ritzels 6, zu gewährleisten, sind die Aufnahmeschlitze des gabelartigen Lagerbocks 8 benötigt. Vergleicht man die Position der Mittelachse 7 in Fig. 2 mit der der Mittelachse in Fig. 1, fällt auf, dass sie aufgrund des Freiheitsgrads der Mittelachse 7 in Höhenrichtung in dem Aufnahmeschlitz gewandert ist.

In Fig. 3 ist das chirurgische Instrument 1 in einem Zustand dargestellt, in dem der gesamte Verfahrweg, also der gesamte Hub, des Schneidelements 23 zurückgelegt ist. So befindet sich das Ritzel 6 am distalen Ende der festen Zahnstange 9.

Eine in Fig. 3 dargestellte Distanz 14 stellt schematisch die zurückgelegte Distanz des Verbindungsteils 11 zwischen dem Ausgangszustand in Fig. 1 und dem Endzustand in Fig. 3 dar. Diese übersteigt die zurückgelegte Distanz der Mittelachse 7, die in etwa der Länge der festen Zahnstange 10 gleicht.

Die feste Zahnstange 9 und die bewegliche Zahnstange 10 sind im Wesentlichen gleich lang ausgestaltet. Somit ist ein minimaler Bauraumbedarf realisiert. Weiterhin birgt dies den Vorteil, dass die beiden Zahnstangen 9, 10 als Gleichteil auszugestalten sind, was die Wirtschaftlichkeit in der Fertigung erhöht.

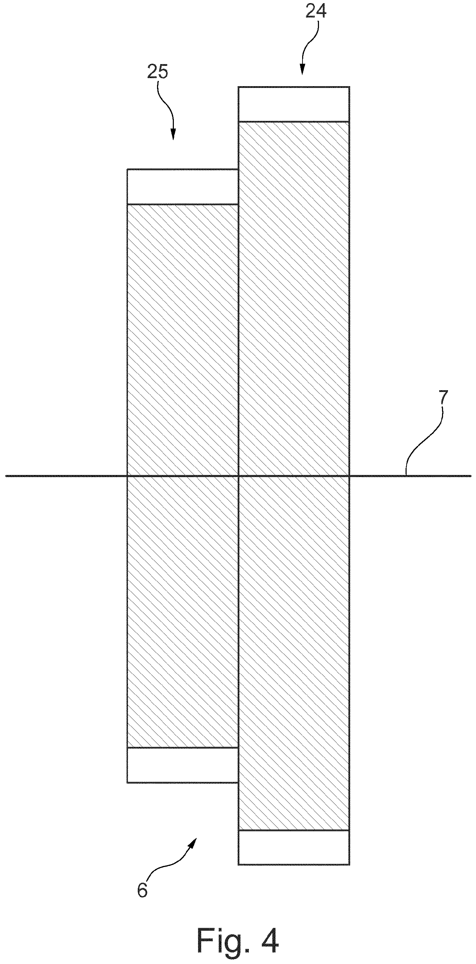

Fig. 4 zeigt ein Ritzel 6 mit zwei Verzahnungsabschnitten 24, 25 unterschiedlichen Durchmessers. Diese Abschnitte 24, 25 können integral oder separat zueinander ausgestaltet sind. Während der Abschnitt 24 dazu vorbereitet ist, in die feste Zahnstange 9 einzugreifen, ist der Abschnitt 25 dazu vorbereitet, in die bewegliche Zahnstange 10 einzugreifen. Dies ermöglicht es, bei Instrumenten 1 mit einem langen Schaft 2 eine ausreichende Verfahrbewegung des Schneidelements 23 zu ermöglichen.

## Patentansprüche

1. Chirurgisches Instrument (1) mit einem Schaftabschnitt (2), der ein axial verfahrbares Schneidelement (23) zumindest teilweise beherbergt oder hält, und mit einem den Schaftabschnitt (2) aufnehmenden Handgriff (3), an dem ein manuell betätigbares Auslöseelement (4) für das Schneidelement (23) beweglich, vorzugsweise verschwenkbar, angeordnet ist,
sowie mit einem Koppelmechanismus (5), der eine Bewegung des Auslöseelements (4) in eine Axialbewegung des Schneidelements (23) wandelt, wobei der Koppelmechanismus (5) ein Ritzel (6) aufweist, das in den vom Koppelmechanismus (5) bereitgestellten Bewegungszug derart zwischengeschaltet ist, dass die Bewegung des Auslöseelements (4) eine Rotation des Ritzels (6) um seine Mittelachse (7) und zusätzlich eine Translationsbewegung der Mittelachse (7) längs der Bewegungsrichtung des Schneidelements (23) selbst bewirkt, wobei
ein Zahnbereich des Ritzels (6) in eine feste Zahnstange (9) eingreift, während ein anderer Zahnbereich des Ritzels (6) in eine bewegliche Zahnstange (10) eingreift, **dadurch gekennzeichnet, dass**
das Ritzel (6) entlang seiner Mittelachse (7) zwei konzentrische Verzahnungsabschnitte (24, 25) unterschiedlichen Durchmessers aufweist, bei dem der eine Verzahnungsabschnitt (24) in die feste Zahnstange (9) eingreift, während der andere Verzahnungsabschnitt (25) in die bewegliche Zahnstange (10) eingreift, und
sich die bewegliche Zahnstange (10) in der Axialrichtung des Schaftabschnitts (2) und zumindest teilweise in Umfangsrichtung um den Schaftabschnitt (2) erstreckt, sodass auch bei einer Rotation des Schaftabschnitts (2) um seine Längsachse das Ritzel (6) in die bewegliche Zahnstange (10) eingreift.

2. Chirurgisches Instrument (1) nach Anspruch 1, **dadurch gekennzeichnet, dass** die Axialbewegung des Schneidelements (23) die von der Mittelachse (7) des Ritzels (6) durchgeführte, von dem Auslöseelement (4) hervorgerufene Translationsbewegung bzw. Axialbewegung der Mittelachse (7) überschreitet.

3. Chirurgisches Instrument (1) nach einem der Ansprüche 1 oder 2, **dadurch gekennzeichnet, dass** die Axialbewegung des Schneidelements (23) im Wesentlichen doppelt so groß ist wie die von der Mittelachse (7) des Ritzels (6) durchgeführte Axialbewegung.

4. Chirurgisches Instrument (1) nach einem der Ansprüche 1 bis 3, **dadurch gekennzeichnet, dass** die von der Mittelachse (7) des Ritzels (6) durchgeführte Axialbewegung im Wesentlichen der Axialbewegung eines Griffbereichs (13) des Auslöseelements (4) entspricht.

5. Chirurgisches Instrument (1) nach einem der Ansprüche 1 bis 4, **dadurch gekennzeichnet, dass** das Ritzel (6) in der Höhenrichtung des chirurgischen Instruments (1) relativ zu dem Auslöseelement (4) frei beweglich ist, während es in der Axialrichtung starr mit dem Auslöseelement (4) gekoppelt ist.

6. Chirurgisches Instrument (1) nach einem der Ansprüche 1 bis 5, **dadurch gekennzeichnet, dass** die feste Zahnstange (9) integral mit dem Handgriff (3) ausgestaltet ist.

7. Chirurgisches Instrument (1) nach einem der Ansprüche 1 bis 6, **dadurch gekennzeichnet, dass** das Ritzel (6) entlang der Umfangsrichtung zumindest zwei konzentrische Verzahnungsgeometrien aufweist, wobei dieselbe Verzahnungsgeometrie, die in die feste Zahnstange (9) eingreift zeitgleich auch in die bewegliche Zahnstange (10) eingreift, sodass mittels einer Schaltbewegung des Ritzels (6) entlang seiner Mittelachse (7) die hervorgerufene Axialbewegung des Schneidelements (23) variierbar ist.

8. Chirurgisches Instrument (1) nach einem der vorstehenden Ansprüche, **dadurch gekennzeichnet, dass** zwischen dem Auslöseelement (4) und dem Ritzel (6) eine zusätzliche Koppeleinheit angeordnet ist, über die eine Relativverschiebung zwischen dem Koppelmechanismus (5) und dem Schneideelement (23) bei unveränderter Stellung des Auslöseelements (4) ermöglicht ist.

9. Chirurgisches Instrument (1) nach einem der vorstehenden Ansprüche, **dadurch gekennzeichnet, dass** der Koppelmechanismus (5) eine Rückholfeder, vorzugsweise nach Art einer Zugfeder, einer Druckfeder oder einer Torsionsfeder, beherbergt, die dazu vorbereitet ist, das Schneidelement (23) nach einer Betätigung in seine Ausgangsposition zurückzuversetzen.

10. Chirurgisches Instrument (1) nach einem der vorstehenden Ansprüche, **dadurch gekennzeichnet, dass** die zwei konzentrischen Verzahnungsabschnitte (24, 25) des Ritzels (6) integral miteinander ausgestaltet sind.

11. Chirurgisches Instrument (1) nach einem der Ansprüche 1 bis 9, **dadurch gekennzeichnet, dass** die zwei konzentrischen Verzahnungsabschnitte (24, 25) als zwei zueinander separate Bauteile ausgestaltet sind und bevorzugt durch einen Spalt voneinander getrennt sind.

12. Chirurgisches Instrument (1) nach einem der vorstehenden Ansprüche, **dadurch gekennzeichnet, dass** der Verzahnungsabschnitt (24) mit dem größeren Durchmesser in die feste Zahnstange (9) eingreift, während der Verzahnungsabschnitt (23) mit dem kleineren Durchmesser in die bewegliche Zahnstange (10) eingreift.

## Claims

1. A surgical instrument (1) comprising a shaft portion (2) which at least partly houses or holds an axially movable cutting element (23), and comprising a handle (3) which receives the shaft portion (2) and on which a manually operable trigger element (4) for the cutting element (23) is arranged movably, preferably pivotably, as well as comprising a coupling mechanism (5) which converts a movement of the trigger element (4) into an axial movement of the cutting element (23), wherein the coupling mechanism (5) has a pinion (6) which is interposed in the train of movement provided by the coupling mechanism (5) such that the movement of the trigger element (4) produces rotation of the pinion (6) about its central axis (7) and additionally a translational movement of the central axis (7) along the direction of movement of the cutting element (23) itself, wherein
a tooth area of the pinion (6) engages in a fixed rack (9), while another tooth area of the pinion (6) engages in a movable rack (10),
**characterized in that**
the pinion (6) includes two concentric toothing portions (24, 25) of different diameters along its central axis (7), with the one toothing portion (24) engaging in the fixed rack (9), while the other toothing portion (25) engages in the movable rack (10) and
the movable rack (10) extends in the axial direction of the shaft portion (2) and at least partly in the circumferential direction around the shaft portion (2) so that the pinion (6) engages in the movable rack (10) even in the case of rotation of the shaft portion (2) about its longitudinal axis.

2. The surgical instrument (1) according to claim 1, **characterized in that** the axial movement of the cutting element (23) exceeds the translational movement or axial movement of the central axis (7) performed by the central axis (7) of the pinion (6) and caused by the trigger element (4).

3. The surgical instrument (1) according to any one of claims 1 or 2, **characterized in that** the axial movement of the cutting element (23) is substantially twice as large as the axial movement performed by the central axis (7) of the pinion (6).

4. The surgical instrument (1) according to any one of claims 1 to 3, **characterized in that** the axial movement performed by the central axis (7) of the pinion (6) substantially corresponds to the axial movement of a grip area (13) of the trigger element (4).

5. The surgical instrument (1) according to any one of claims 1 to 4, **characterized in that** the pinion (6) is freely movable in the height direction of the surgical instrument (1) relative to the trigger element (4), while in the axial direction it is rigidly coupled to the trigger element (4).

6. The surgical instrument (1) according to any one of claims 1 to 5, **characterized in that** the fixed rack (9) is configured to be integral with the handle (3).

7. The surgical instrument (1) according to any one of claims 1 to 6, **characterized in that** the pinion (6) has at least two concentric toothing geometries along the circumferential direction, wherein the same toothing geometry which engages in the fixed rack (9) simultaneously also engages in the movable rack (10) so that the caused axial movement of the cutting element (23) is variable by means of a switching movement of the pinion (6) along its central axis (7).

8. The surgical instrument (1) according to any one of the preceding claims, **characterized in that** between the trigger element (4) and the pinion (6) an additional coupling unit is arranged, through which a relative displacement between the coupling mechanism (5) and the cutting element (23) is made possible while the position of the trigger element (4) remains unchanged.

9. The surgical instrument (1) according to any one of the preceding claims, **characterized in that** the coupling mechanism (5) houses a return spring, preferably of the type of a tension spring, a compression spring or a torsion spring, which is prepared to return the cutting element (23) to its home position after actuation.

10. The surgical instrument (1) according to any one of the preceding claims, **characterized in that** the two concentric toothing portions (24, 25) of the pinion (6) are formed to be integral with each other.

11. The surgical instrument (1) according to any one of claims 1 to 9, **characterized in that** the two concentric toothing portions (24, 25) are configured as two components separate from each other and are preferably separated from each other by a gap.

12. The surgical instrument (1) according to any one of the preceding claims, **characterized in that** the toothing portion (24) having the larger diameter engages in the fixed rack (9), while the toothing portion (23) having the smaller diameter engages in the movable rack (10).

## Revendications

1. Instrument chirurgical (1) avec une section fourreau (2), qui accueille ou tient au moins partiellement un élément coupant déplaçable axialement (23), et avec une poignée (3) logeant la section fourreau (2), poignée au niveau de laquelle un élément déclencheur actionnable manuellement (4) est disposé pour l'élément coupant (23) de manière mobile, de préférence pivotante,
ainsi qu'avec un mécanisme de couplage (5) qui transforme un mouvement de l'élément déclencheur (4) en un mouvement axial de l'élément coupant (23), dans lequel le mécanisme de couplage (5) présente un pignon (6) qui est intercalé dans la traction de mouvement mise à disposition par le mécanisme de couplage (5) de telle sorte que le mouvement de l'élément déclencheur (4) exerce de lui-même une rotation du pignon (6) autour de son axe central (7) et en outre un mouvement de translation de l'axe central (7) le long de la direction de mouvement de l'élément coupant (23), dans lequel
une zone de dent du pignon (6) s'engrène dans une crémaillère fixe (9), pendant qu'une autre zone de dent du pignon (6) s'engrène dans une crémaillère mobile (10),
**caractérisé en ce que**
le pignon (6) présente le long de sont axe central (7) deux sections d'engrenage concentriques (24, 25) de diamètre différent, dans lequel l'une section d'engrenage (24) s'engrène dans la crémaillère fixe (9) pendant que l'autre section d'engrenage (25) s'engrène dans la crémaillère mobile (10), et
la crémaillère mobile (10) s'étend dans la direction axiale de la section fourreau (2) et au moins partiellement dans la direction périphérique autour de la section fourreau (2), de sorte que le pignon (6) s'engrène également lors d'une rotation de la section fourreau (2) autour de son axe longitudinal dans la crémaillère mobile (10).

2. Instrument chirurgical (1) selon la revendication 1, **caractérisé en ce que** le mouvement axial de l'élément coupant (23) dépasse le mouvement de translation ou le mouvement axial de l'axe central (7) provoqué par l'élément déclencheur (4) et réalisé par l'axe central (7) du pignon (6).

3. Instrument chirurgical (1) selon l'une quelconque des revendications 1 ou 2, **caractérisé en ce que** le mouvement axial de l'élément coupant (23) est pour l'essentiel le double du mouvement axial réalisé par l'axe central (7) du pignon (6).

4. Instrument chirurgical (1) selon l'une quelconque des revendications 1 à 3, **caractérisé en ce que** le mouvement axial réalisé par l'axe central (7) du pignon (6) correspond pour l'essentiel au mouvement axial d'une zone de préhension (13) de l'élément déclencheur (4).

5. Instrument chirurgical (1) selon l'une quelconque des revendications 1 à 4, **caractérisé en ce que** le pignon (6) se déplace librement dans la direction de la hauteur de l'instrument chirurgical (1) par rapport à l'élément déclencheur (4), pendant qu'il est couplé de manière rigide à l'élément déclencheur (4) dans la direction axiale.

6. Instrument chirurgical (1) selon l'une quelconque des revendications 1 à 5, **caractérisé en ce que** la crémaillère fixe (9) est configurée d'un seul tenant avec la poignée (3).

7. Instrument chirurgical (1) selon l'une quelconque des revendications 1 à 6, **caractérisé en ce que** le pignon (6) présente le long de la direction périphérique au moins deux géométries d'engrenage concentriques, dans lequel la même géométrie d'engrenage qui s'engrène dans la crémaillère fixe (9) s'engrène simultanément également dans la crémaillère mobile (10), de sorte que, au moyen d'un mouvement d'accouplement du pignon (6) le long de son axe central (7) le mouvement axial provoqué de l'élément coupant (23) est variable.

8. Instrument chirurgical (1) selon l'une quelconque des revendications précédentes, **caractérisé en ce que,** entre l'élément déclencheur (4) et le pignon (6), une unité de couplage supplémentaire est disposée, par l'intermédiaire de laquelle un déplacement relatif entre le mécanisme de couplage (5) et l'élément coupant (23) dans une position de l'élément déclencheur (4) non modifiée est rendu possible.

9. Instrument chirurgical (1) selon l'une quelconque des revendications précédentes, **caractérisé en ce que** le mécanisme de couplage (5) accueille un ressort de maintien, de préférence du type ressort de traction, ressort de compression ou ressort de torsion, qui est préparé pour ramener l'élément coupant (23) dans sa position initiale après un actionnement.

10. Instrument chirurgical (1) selon l'une quelconque des revendications précédentes, **caractérisé en ce que** les deux sections d'engrenage concentriques (24, 25) du pignon (6) sont configurées d'un seul tenant l'une avec l'autre.

11. Instrument chirurgical (1) selon l'une quelconque des revendications 1 à 9, **caractérisé en ce que** les deux sections d'engrenage concentriques (24, 25) sont configurées en tant que deux composants séparés l'un de l'autre et de préférence séparées l'une de l'autre par une fente.

12. Instrument chirurgical (1) selon l'une quelconque des revendications précédentes, **caractérisé en ce que** la section d'engrenage (24) s'engrène dans la crémaillère fixe (9) avec le plus grand diamètre pendant que la section d'engrenage (23) s'engrène dans la crémaillère mobile (10) avec le plus petit diamètre.
